# EUROPEAN PATENT APPLICATION

(11) **EP 4 376 019 A1**
(43) Date of publication of application: **29.05.2024**
(21) Application number: 22209742.0
(22) Date of filing: 25.11.2022
(51) Int. Cl.: G16H 40/63, G16H 50/20

(54) **SYSTEMS AND METHODS FOR ASSISTING INDIVIDUALS TO ACCESS EYE HEALTH EXAMINATIONS**

(71) Applicant: Ocumeda AG, 8586 Riedt bei Erlen (CH)
(72) Inventor: Kirr, Jörg-Christian, 4588 Unterramsern (CH); Haarburger, Christoph, 81927 München (DE); Wiechers, Benedikt, 80634 München (DE)
(74) Representative: BOVARD AG

(57) **Abstract**

The present invention relates to a system for providing eye health examinations, comprising: a plurality of diagnostic centres, wherein the diagnostic centres are configured to transmit requests to a network of ophthalmologist to administer eye examinations for customers; receive instructions over the network to remotely control operation of ophthalmic equipment in order to administer one or more tests pertaining to the eye examinations; and generate customer examination data pertaining to the one or more tests administered using the ophthalmic equipment. In addition, the system comprises a plurality of practitioner devices associated with ophthalmologists, wherein the practitioner devices are configured to receive the customer examination data in response to accepting one or more of the requests associated with administering the eye examinations; display the received customer examination data; and generate customer evaluation data associated with the one or more accepted requests; at least one server configured to in response to receiving the requests from the diagnostic centres, analyse practitioner data to select ophthalmologists for handling the requests; receive the customer examination data from the diagnostic centres; transmit the customer examination data to the practitioners devices associated with the selected ophthalmologists; receive customer evaluation data from the practitioner devices associated with the selected ophthalmologists. Eye health reports based, at least in part, on the selected ophthalmologists' review and evaluation of the customer examination data are then provided to the customers.

## Description

### Field of the invention

The current invention relates to the field of telehealth, in particular to the field of ophthalmology and the conduct of eye health checkups i.e. screenings. Certain representations are addressed, in particular, to systems and methods for supporting individuals in obtaining eye health assessments through a diagnostic centre that comprises ophthalmic equipment and devices for conducting such tests and procedures relevant to the customers' eye health. Individuals with a normal eye health perception are examined with the aim to separate those who have normal findings from those who have an undiagnosed eye disease or disorder. Trained personnel at a diagnostic centre at different health providers perform various eye health test and measurements with help of diagnostic ophthalmic equipment and devices. According to these representations, the diagnostic centre uploads the obtained data about the customers and the tests via a browser-based software solution to a distant ophthalmologist for review and evaluation. The subsequent initial assessment of the measurement results is carried out by a network of ophthalmologists and is partially assisted by an artificial intelligence solution. The remote ophthalmologist sends the customer an eye health report. The aim is to refer customers/patients in case of pathological findings to a local ophthalmologist of their choice for further diagnostics and treatment.

### Background of the invention

Individuals are nowadays more likely to visit their physician for a variety of checkups/screenings, thanks to greater knowledge of health monitoring, prevention, and early identification of various medical disorders and diseases. In general, individuals should see a medical specialist on a regular basis for the monitoring, diagnosis, and treatment of medical conditions in a variety of areas, including cardiovascular health, diabetes and metabolic disorders monitoring, and various cancer screening.

The inspection of an individual's eyes is one area where regular visits to a medical specialist are very important. Typically, an eye check-up includes various tests for diagnosing and monitoring eye problems such macular degeneration, diabetic retinopathy, glaucoma, cataracts among others.

Accessing eye health assessments on a regular and ongoing basis has a number of significant benefits. Regular visits and checks by an individual, for example, enable ophthalmologist to monitor and track the health of the individual's eyes, as well as detect and diagnose specific abnormalities, diseases, and other changes, just as they do with many other sorts of medical tests.

Given these and other benefits of regular eye examinations, it's no surprise that several well-known ophthalmologic societies and organizations recommend that people see ophthalmologist once every one to two years. This is important because it enables for early detection and furthermore for diagnosis, and treatment of many disorders, which increases the chances of a successful therapy. In fact, when recognized and identified early enough, many ailments and diseases are often curable or even prevented. Furthermore, it is generally recognized that visual alterations can occur unexpectedly. Eyesight can degrade continuously over time, such as at particular times in a person's life.

Furthermore, regular eye exams are especially important for certain people, such as those who have a higher risk of developing various disorders and diseases due to their demographics or other characteristics, such as age, race, profession, personal and/or family history of diseases or disorders, etc.

Comprehensive eye examinations, in particular, may involve some or all of the following tests and procedures (or tests and procedures of a similar nature):
i. objective and subjective refraction and/or other tests to check visual acuity;
ii. slit lamp examination using biomicroscopy;
iii. examination of the external eye, examination of the extraocular muscles; peripheral vision test (e.g., by checking the visual field by confrontation); examination of the pupils; colour vision test; test for stereopsis for depth perception; cover test for strabismus;
iv. examination of eyelids, conjunctiva, cornea, anterior chamber, iris and lens;
v. measure eye pressure and/or intraocular pressure;
vi. examination of macula, vessels, optic nerve, peripheral retina, and vitreous humour and imaging of fundus using a fundus camera;
vii. optical coherency tomography (OCT) scan; or
viii. evaluation of central vision (e.g., using the Amsler grid);
ix. in some circumstances, administering specific tests to patients for specific diseases or disorders may be beneficial. (e.g., diabetical retinopathy, glaucoma, macular degeneration or hypertensive retina such as where there is individual and/or family history of diseases or disorders).

Despite the fact that frequent eye health check-ups are proven to be beneficial, many people only see an ophthalmologist and get their eyes examined on an irregular basis. Others do not see an ophthalmologist at all, or only do so when they have a medical condition or notice a potential vision problem. Unfortunately, eye diseases develop oftentimes unnoticed and when detected to late a substantial uncurable damage already occurred.

In fact, 75 to 80 % of the Swiss or German adults are estimated not to get their eyes examined at all or just once every five years or more.

Furthermore, even among those who have eye tests on a more regular basis, the examinations they receive frequently do not meet the standards of a comprehensive eye examination. Many people, for example, may only receive a vision check and/or a limited set of visual acuity tests when they visit a general practitioner for driving assessments or an optician for their glass correction and lull themselves in a false security because they do not have the medical understanding that an ophthalmologist is needed to give an assessment if there are eye diseases or disorders.

Despite the fact that there are numerous contributing factors, the key reasons why many people fail to have their eyes examined on a regular basis are time, expense, and convenience. Typically, in order to obtain an eye examination, a person must devote time and effort to locate, choose, and schedule an appointment with an ophthalmologist. As a result, time is spent going to and from the ophthalmologist office, as well as waiting for him to arrive.

Similarly, the number of patients who can be seen and examined by an ophthalmologist is restricted by a variety of factors, including the time required to evaluate each patient, update the patient's records, and prepare equipment.

As a result, people are frequently forced to schedule appointments at inconvenient times and/or drive to other, less convenient locations to see an ophthalmologist. In order to see as many patients as possible, ophthalmologists may limit the number of tests and treatments performed, as well as the time spent performing them, lowering the amount of time required for each patient.

Furthermore, the expense associated with seeing an ophthalmologist might deter, if not outright prevent, many people from getting regular eye exams. This is made worse by the fact that health insurances do not cover an eye screening and individuals must pay some or all of the fees out of pocket. (e.g. in Germany). In summary, seeing an ophthalmologist on a regular basis can be a time-consuming, inconvenient, and a costly commitment.

As a result, there is a need for alternative techniques to providing eye check-ups and screenings that reduce the amount of time and money required by individuals and so motivate them to have regular exams.

In the past, there have been several attempts to develop systems that would simplify and automate the eye examination process. These previous systems, on the other hand, have a variety of flaws and restrictions that have prevented them from gaining widespread customer acceptance.

As a downside, many of these devices simply offer a vision or visual acuity testing. Many of these systems, therefore, are restricted to a limited or incomplete set of procedures and tests, and do not provide access to a comprehensive eye check-up. Another disadvantage is that many of these systems necessitate the presence of an on-site ophthalmologist.

Other methods, on the other hand, do not allow for any input or feedback from an ophthalmologist. Another disadvantage is that none of these methods allow the examinee to communicate with a remote ophthalmologist in real time. Another disadvantage is that many of these systems lack the ability to detect or diagnose suspected illnesses, abnormalities, or risk factors automatically.

As a result, an eye testing and evaluation system for providing eye health assessments to individuals is required.

There is also a need for a system that allows people to have eye exams through a user-friendly diagnostic centre that offers a variety of services.

There is also a need for a system that allows people to get a comprehensive eye examination. The system at a diagnostic centre should work in an automated way, allowing individuals to get eye health screenings with no on-site help from an ophthalmologist.

A system that provides an interface between the diagnostic centre and a remote ophthalmologist across a network is required to allow an ophthalmologist to assess an individual's eye health.

There is further a need for a system that enables the remote ophthalmologist to assess a person's eye health and provides the seeker with eye health reports and/or recommendations from the remote ophthalmologist.

There is still further a need for a system that permits a remote ophthalmologist or offshore technician to control or monitor the ophthalmologic testing equipment and/or the administration of various eye health tests.

There is further a need for a system that enables the remote ophthalmologist to assess the data more time-efficient by assisting the ophthalmologist with an artificial intelligence (Al) solution.

There is further a need for an artificial intelligence (Al) assisted solution that supports the ophthalmologist in the diagnostic workflow, because the demographic changes in the future will lead to both more eye diseases as well as fewer ophthalmologists.

There is also a need for a system that encourages individuals to have frequent eye health assessments by decreasing the time and cost involved.

There is also a need for a system that sensitizes health insurers that costs for the eye screening are covered by health insurances.

There is also a requirement for a system that can be accessible by individuals from a variety of convenient locations.

The present invention addresses these and other needs in an advantageous manner.

### Summary of the invention

More specifically, the current invention in the field of ophthalmology is set up to conduct eye health check-ups.

It supports individuals in obtaining eye health assessments at a Checkup Point diagnostic centre (CuPDC) that comprises ophthalmic equipment and devices for performing tests and procedures relevant to the customers' eye health. The CuPDC can be located at different players in the health sector (e.g. general practitioners, endocrinologist, pharmacies, opticians etc.) From a CuPDC relevant data can be transmitted via a secured and encrypted platform solution (browser based) to an ophthalmologist at the Checkup Point reading centre (CuPRC). The ophthalmologist provides a review and evaluation and sends the customer an eye health report.

In the present invention, a system is provided that includes a CuPDC that is configured to create customer examination data relevant to an examination of a customer's eye, according to certain embodiments. A user interface for receiving input and presenting information to a consumer is provided by the customer diagnostic centre. The CuPDC also includes ophthalmic equipment for administering tests to customers, as well as an equipment controller for controlling the ophthalmic equipment's operation. A diagnostic centre server is configured to receive client examination data from the CuPDC across a network and to allow an ophthalmologist at the CuPRC to view that data. The ophthalmologist's practitioner device is set up to receive at least a portion of the customer examination data from the diagnostic centre server and display it to the ophthalmologist. The ophthalmologist's assessment and evaluation of the client examination data generates customer evaluation data. The customer receives an eye health report via the network.

In addition, according to the present invention, a method for eye health examinations is offered. Customer examination data pertaining to an examination of a customer's eye at a CuPDC is generated as part of the procedure. A user interface is included in the CuPDC for receiving and giving information to the customer. Ophthalmic equipment for administering tests to the client and an equipment controller configured to govern the operation of the ophthalmic equipment are also included in the CuPDC. Customer examination data is received by a diagnostic centre server over a computer network from the CuPDC. An ophthalmologist at the CuPRC can access the consumer examination data via the diagnostic centre server. The diagnostic centre server sends the client examination data to a device to get assessed by the ophthalmologist. Customer feedback data is generated regarding the ophthalmologist's study and evaluation of the customer examination data. The client receives an eye health report via the network that is based on the customer evaluation data.

A server is configured to deliver services related with eye health assessments. Customer examination data pertaining to an examination of a customer's eye administered at a CuPDC is received by the server across a computer network. A user interface is included in the CuPDC for receiving and giving information to the customer. Ophthalmic equipment for administering tests to customers is also included in the customer diagnostic centre, as well as an equipment controller for controlling the ophthalmic equipment's operation. The server is set up to allow an ophthalmologist to access customer examination data and to send customer examination data from the diagnostic centre server to a device linked with the ophthalmologist at the CuPRC. Customer assessment data pertaining to the ophthalmologist's study and evaluation of the customer examination data is also received by the server. The client receives an eye health report via the network that is based, at least in part, on the customer evaluation data.

In addition, according to the present invention, a system is configured to provide eye health examinations. The system includes a number of CuPDCs that are configured to send requests for ophthalmologists at the CuPRC to administer eye examinations to customers over a network and to receive instructions for remotely controlling ophthalmic equipment to administer one or more tests related to the eye examinations over the network. Customer examination data referring to one or more tests done using the ophthalmic equipment is also generated by the CuPDCs. A plurality of devices associated with ophthalmologists are also included in the system, each of which is configured to receive and display the customer examination data in response to accepting one or more of the requests, as well as to generate customer evaluation data associated with the one or more accepted requests. At least one server is also part of the system. The server analyses practitioner data in response to receiving requests from CuPDCs and selects ophthalmologists at the CuPRC to handle the requests. The practitioner data may include availability data, which indicates whether the ophthalmologists are currently connected to the system and available to administer eye examinations, scheduling data, which indicates days and hours when the ophthalmologists are available to handle requests, and prior customer data, which identifies customers who have previously used the ophthalmologists for eye examinations. The server is set up to accept client examination data from diagnostic facilities and send it to the devices linked with the chosen ophthalmologists. Customer assessment data from the devices linked with the designated ophthalmologists is also received by the server. Eye health reports based, at least in part, on the selected ophthalmologist' review and evaluation of the customer examination data are provided to the customers.

A server is configured to provide services connected to eye health assessments. The server receives a request from a CuPDC across a network to select an ophthalmologist at the CuPRC to perform an eye examination on a customer. The server evaluates practitioner data in response to the request and selects an ophthalmologist to handle the request. The practitioner data includes availability data, which shows which ophthalmologists are currently available to administer the eye examination, scheduling data, which shows which days and hours ophthalmologists are available to handle requests, and prior customer data, which shows which ophthalmologists have previously administered eye examinations to the customer. The server is also set up to receive customer examination data generated by ophthalmic equipment at the CuPDC while administering one or more eye examination tests, as well as to transmit customer examination data to the practitioner device associated with the chosen ophthalmologist. The practitioner device linked with the chosen ophthalmologist at the CuPRC receives customer evaluation data. The customer is given an eye health report that is based on the selected ophthalmologist's study and evaluation of the customer examination data.

These and other features and advantages will become apparent from the following detailed description of illustrative embodiments thereof, which is to be read in connection with the accompanying drawings.

### Brief description of the drawings

The inventive principles are illustrated in the figures of the accompanying drawings which are meant to be exemplary and not limiting, in which like references are intended to refer to like or corresponding parts, and in which:
Figure 1 is a pictorial diagram of an eye testing and evaluation system in accordance with certain embodiments of the present invention; and
Figure 2 is a flow chart illustrating an exemplary workflow for embedding a triage AI model into the reporting workflow.

### Detailed description of embodiments of the invention

The accompanying drawings, which form a part of this document and in which is shown by way of illustration certain embodiments in which the invention may be practiced, are referred to in the following description. Other embodiments and structural modifications may be used, as long as they do not depart from the scope of the current invention.

Eye health examinations are well-known to provide numerous important benefits, and as a result, virtually all reputable associations and professionals in the field of ophthalmology strongly advise that individuals receive such examinations and screenings on a regular and consistent basis. Despite this, many people either do not have their eyes examined at all, or only do so intermittently or in response to an emergency medical problem or condition. Many people find it difficult to get regular eye exams because of the significant time, money, and inconvenience involved in locating an eye care professional, scheduling an appointment, and visiting the expert's office to have the examination. To encourage such individuals and others to have regular eye health examinations and vision examinations, systems and methods are provided herein that allow individuals to obtain eye health examinations and vision examinations through Checkup Point diagnostic centres (CuPDCs) at a variety of convenient locations, thereby reducing the time and expense incurred by such individuals.

The current invention, in some implementations, relates to an eye testing and evaluation system. It includes devices, basic hardware components, computer hardware and software for offering eye health examinations to clients (i.e., anyone who wants eye health assessments). Customers may receive eye health examinations at a Checkup Point diagnostic centre (CuPDC) that includes various ophthalmic and/or vision testing equipment and instruments for administering a variety of tests and procedures and collecting various data pertaining to the customers' eye health. I n certain embodiments, a network connects the Checkup Point diagnostic centre (CuPDC) to an ophthalmologist. Various data, including data pertaining to a customer and data related with the eye health tests and procedures administered to the customer, is supplied to the ophthalmologist for analysis and/or confirmation according to certain of these embodiments. In other embodiments, the ophthalmologist sends the client an eye health report, recommendations, and/or referrals based on the customer's and testing results. Certain implementations allow an ophthalmologist or an off-site technician to operate or monitor the diagnostic centre's ophthalmic testing equipment, as well as the administration of various tests, via a remote equipment interface.

The eye testing and evaluation system, associated arrangements and systems, apparatuses, and methods described below address many of the current barriers and limitations in administering eye health examinations and vision examinations to individuals, and encourages individuals to undergo such examinations and examinations on a regular and consistent basis by providing incentives.

### System Architecture for Eye Testing and Evaluation

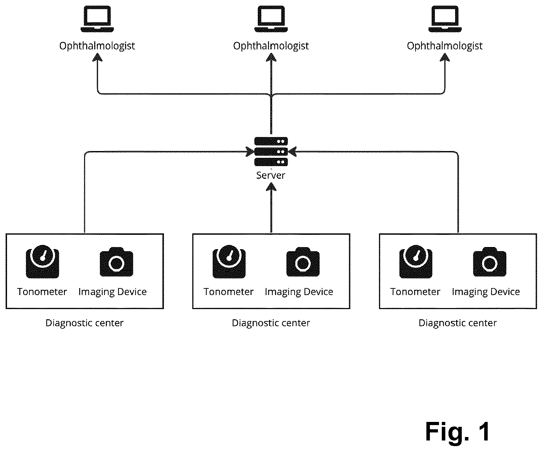

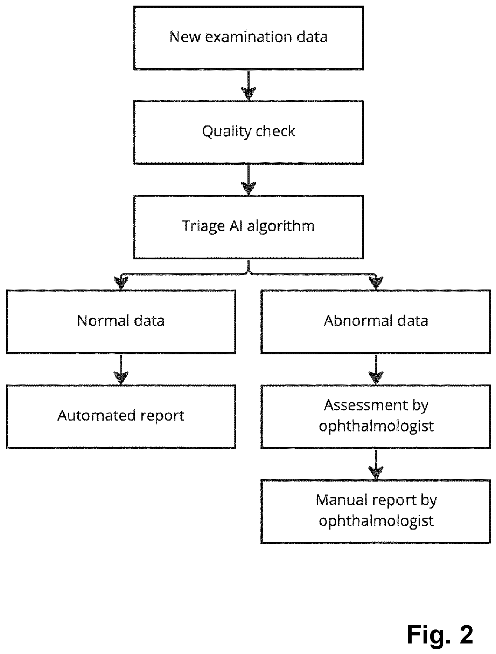

The eye testing and evaluation system may be supplied in certain embodiments through any suitable form of general hardware components and devices, computer hardware and software, or a combination of any of the foregoing that allows consumers to acquire eye health examinations. Comprehensive examinations and/or screenings may be included in eye health examinations. In accordance with some embodiments, exemplary illustrations of the architecture of the eye testing and evaluation system and accompanying apparatuses, systems, and components are illustrated in Figures 1 and 2 and explained below. The eye testing and evaluation system may include different components and subcomponents, as shown in Figures 1 and 2, such as apparatuses, equipment, servers, processors, networks, and personal devices.

It is acknowledged that the components illustrated may be logical components, and that the terminology used to describe each component is for illustration purposes only and should not be interpreted as restricting. Each component and subcomponent may comprise the necessary apparatuses, devices, and computer hardware, software, and firmware to enable data collection, processing, storage, communication, presentation, and/or reception. One or more electrical components, mechanical components, instruments, computer processors, computer servers, data stores, storage media, memory, and other components or subcomponents may make up a component or subcomponent. One or more executable computer-readable instructions received via a computer-readable storage medium may direct the functionality of a component. A processor may be used to carry out one or more functions based on instructions, programs, or procedures stored in the processor or another memory source. Any apparatus capable of storing data, such as computer programs, instructions, and other necessary data, is referred to as memory. One or more interfaces may be included to allow data to be presented, manipulated, transmitted, and received. One or more networks or physical links may be used to enable data communication. One or more networks or physical links may be used to enable data communication. For security reasons, all data exchanged by or between components may be encrypted (e.g., with a 256-bit advanced encryption algorithm). A network may consist of one or more wide-area networks (WANs) (such as the Internet), local area networks (LANs), wireless local area networks (WLANs), a mobile wireless network, a combination of any of the foregoing, and/or any other suitable networks, as well as any component (physical or logical) required for utilizing a network's functionality, such as routers, adapters, subnets, and/or any other suitable networks.

In accordance with some embodiments, Figure 1 shows a graphical schematic of an eye testing and evaluation system 100. The system includes a Checkup Point diagnostic centre (CuPDC) with different components, such as general hardware, mechanical devices, electronic equipment, computer hardware and software, and other suitable components for allowing customers to undergo eye health examinations, as illustrated in Figure 1. Checkup Point diagnostic centre (CuPDC) may incorporate a variety of ophthalmic equipment and tools, such as refractors, tonometers, biomicroscopes, and fundus cameras, to administer a variety of tests and procedures to consumers in certain implementations. Checkup Point diagnostic centre (CuPDC) may contain one or more interfaces, such as a customer interface, for receiving customer inputs, selections, answers, and other data and outputting different information to the customers. Checkup Point diagnostic centre (CuPDC) may also comprise a network interface for interacting with other systems and devices over one or more networks, such as network 50 in Figure 1. Checkup Point diagnostic centre (CuPDC) may comprise an operator interface that allows an onsite technician or operator to monitor or manage ophthalmic equipment and instruments (or a portion thereof) and/or the administration of tests to custom-educated patients.

Checkup Point diagnostic centre (CuPDC) may contain computer hard-ware, software, or a mix of the two, such as processors, memory, controllers, applications, and other suitable computing devices and components that control Checkup Point diagnostic centre (CuPDC) overall operation. These components may, for example, make it easier to provide eye health exams, analyse data from customers and operators as well as data generated by ophthalmic equipment and instruments, and/or manage data flow to and from other systems and devices through a network. Computer hardware and software, as well as the devices, equipment, and other components that may be included in and/or used by Check-up Point diagnostic centre (CuPDC) are illustrated and described in greater detail below in connection with Figures 1 and 2.

Checkup Point diagnostic centre (CuPDC) may be provided as a self-contained structure, such as a booth or other suitable enclosure, in some embodiments. A variety of structures, each of which may incorporate certain components of Checkup Point diagnostic centre (CuPDC), may be provided in some of these embodiments. Checkup Point diagnostic centre (CuPDC) may be large enough to allow customers to enter and/or sit down in some of these embodiments. Checkup Point diagnostic centre (CuPDC) may be supplied in a free-standing form, such as a terminal, kiosk, or the like, or may be included within one or more existing facilities.

Checkup Point diagnostic centre (CuPDC) can be located and operated in a variety of settings. Checkup Point diagnostic centre (CuPDC) may, for example, be provided at residential homes for the elderly, office buildings or various shopping places, such as retail stores, malls, event centres, or other indoor areas in some implementations. Checkup Point diagnostic centre (CuPDC) may be housed in a retail store, office, or the like that is associated with providing one or more eye care products or services, such as an optician's, optometrist' s, general practitioner's/endocrinologist's office in some of these embodiments.

Checkup Point diagnostic centre (CuPDC) could, for example, be located in a variety of outdoor places and surroundings, such as outlet malls, or town centres. A diagnostic centre may be provided at the home or office of a client or individual seeking an examination in some embodiments. Checkup Point diagnostic centre (CuPDC) may be provided at almost any place or setting suited for permitting customers to access Checkup Point diagnostic centre (CuPDC) and get eye-health assessments in various embodiments. Checkup Point diagnostic centre (CuPDC) may be provided as a fixed building or device in some implementations, or it may be built to be portable in others.

### Checkup Point diagnostic centre (CuPDC)

As previously stated, the eye testing and evaluation system's client diagnostic centres can be implemented using a variety of structures and physical arrangements. Checkup Point diagnostic centre (CuPDC) may also comprise a variety of components, such as general hardware components, mechanical devices, electrical equipment, computer hardware and software, to enable clients to get eye health assessments through the Checkup Point diagnostic centre (CuPDC). It should be understood that the components described below are intended to be illustrative only, and that other components may be included with the Checkup Point diagnostic centre (CuPDC) that are provided in connection with the eye testing and evaluation system, corresponding arrangements, systems, and methods described herein.

Checkup Point diagnostic centre (CuPDC) includes, in certain embodiments, processor, memory, equipment controller, vision examination system, eye health examination system, customer interface, onsite operator interface, and network interface. Processor may be in charge of overseeing the entire operation of Checkup Point diagnostic centre (CuPDC), as well as controlling part or all of its components. Processor can be a single processing unit, such as a CPU or microcontroller, or a computing device, such as a personal computer or workstation, in some embodiments. Other embodiments are possible. Multiple processing units, computer devices, or any mix of these could make up processor. Software, programs, and/or instructions performed by processor (or other components) and/or data generated, received, and used by various components of Checkup Point diagnostic centre (CuPDC) may be stored and retrieved in memory. Any known type of data storage device and/or media, such as magnetic media, optical media, random access memory, read-only memory, data cache, and so on, may be included in Memory, as well as any combination of one or more data storage devices and media. Memory (or a portion of it) may be integrated with one or more additional components of Checkup Point diagnostic centre (CuPDC), such as CPU, in some embodiments.

Various ophthalmic devices, equipment, and instruments (e.g., refractors, tonometers, fundus cameras etc.) and/or other associated systems, devices, components, and/or computer hardware, software, and data that allow customers to receive vision examinations at Checkup Point diagnostic centre (CuPDC) may be included in vision examination system. In some of these implementations, vision examination system is used to administer a variety of tests and procedures to customers, as well as to measure, capture, and/or generate data related to identifying the customers' visual acuity and/or analysing certain other characteristics of their vision. Similarly, Checkup Point diagnostic centre (CuPDC) may incorporate various ophthalmic equipment (e.g., tonometers, fundus camaras, biomicroscopes, etc.) and/or other associated components that allow clients to get eye health examinations. Eye health examination system may be utilized in connection with providing a variety of tests and treatments to customers, as well as measuring, capturing, and/or creating different data related to evaluating the customers' eye health in some of these embodiments.

As another example, after a customer has received an eye health examination and/or a vision examination from Checkup Point diagnostic centre (CuPDC) (or another Checkup Point diagnostic centre (CuPDC)), customer interface may display various information about the tests, such as results, diagnoses, recommendations, and other data generated automatically by Checkup Point diagnostic centre (CuPDC) (e.g., eye-health reports, recommendations, referrals).

### Vision Examination System

As previously stated, some or all of the Checkup Point diagnostic centre (CuPDC) offered with the eye testing and evaluation system may contain a vision examination system in certain implementations. The vision examination system may include a variety of ophthalmic devices, equipment, and instruments, as well as other general hard-ware, mechanical and electronic devices, and/or computer software and hardware used by the Checkup Point diagnostic centre (CuPDC) to conduct vision examinations. Vision examination system, for example, may contain auto-phoropter, lens houser, auto refractor, lensometer, eye chart, and vision examination controller.

### Eye Health Examination System

As previously stated, in some implementations, an eye health examination system may be included in some or all of the client diagnostic centres offered with the eye testing and evaluation system. The Checkup Point diagnostic centre (CuPDC) may use various ophthalmic devices, equipment, and tools, as well as other general hardware, mechanical and electrical devices, and/or computer software and hard-ware, to conduct eye health examinations to customers. Eye health examination system, for example, may contain digital imager, biomicroscope, fundus camera, tonometer, and eye health examination controller.

In certain cases, fundus camera could be any camera or other similar equipment that allows for improved seeing and/or digital photography of the back portion of a person's eyes. The vitreous, choroids, retina, macula, and/or optic nerve of a person's eyes, for example, might be detected and photographs captured using fundus camera. It's also possible to utilize a tonometer. Any acceptable equipment or gadget that may be used to measure the intraocular pressure of a person's eyes, such as to detect, diagnose, and/or evaluate the potential of glaucoma, is included in tonometer. Tonometer, for example, may allow intraocular pressure to be measured in millimeters of mercury (mmHg) in certain of these implementations by sensing the degree of indentation.

Pachymeters/Tomographers-an ultrasound pachymeter, optical coherence tomographer (OCT), optical coherence pachymeter (OCP), computerized corneal topographer, corneal waveform device (CWF), anterior segment optical coherence tomographer, and/or ultrasonic biomicroscope may be incorporated. These and other devices may be utilized in some embodiments to take measurements and perform various tests related to the cornea, optic nerve, and/or retina, as well as to examine the anatomic relationship between the lens, iris, and cornea. (For example, the depth of the anterior chamber and the structures behind the iris, the position of the ciliary body, or the cyclodialysis cleft). Confocal microscopy, ultrasound, optical biometry with a camera, or an OCT and online OCP can all be used to non-invasively evaluate corneal thickness. An ultrasonic transducer can be used to touch the cornea and/or a CWF can be used to acquire an ultra-high definition echogram instead (or in addition). Low coherence interferometry can be used with OCT to assess the thickness of the optic nerve and the retinal nerve fiber layer (RNFL) (e.g., assessed in the peripapillary area using circular scans centreed on the optic nerve head (ONH)). RNFL thickness measurements may be shown in a TSNIT orientation in some of these embodiments and compared to age-matched controls. These devices and tests may allow the system to track changes in a customer's corneas, intraocular pressure, ONH, and retina, analyse changes in eye tissues, and/or count endothelial cells and detect corneal abnormalities in some embodiments. These devices and tests may be utilized to confirm diagnoses and monitor results following various types of interventions in some of these embodiments (e.g., to detect wrinkling of chorioretinal folds in the posterior pole that are difficult to assess using direct or indirect ophthalmoscopy).

Fundus/Fundus cameras-one or more fundus and/or fundus cameras, such as mydriatic and non-mydriatic fundus cameras, hybrid digital mydriatic/non-mydriatic fundus cameras, non-mydriatic fundus cameras, and autoflourescense cameras, may be incorporated. These and other devices may be utilized for fundus photography with and/or without dilated pupils to create images of the eye's internal surface, including the retina, optic disc, macula, and posterior pole, in some embodiments. These images may be utilized to analyse the posterior of the eye and disorders or abnormalities of the retina, choroid, vitreous, optic disc, macula, and posterior pole in some of these embodiments.

Heidelberg Retina Tomography (HRT)-This technique uses lasers to scan several cross-section images in order to construct and analyse a three-dimensional depiction of the optic nerve. The 3-D representation may be used to examine thickness, surfaces of the optic cup, cup-to-disc ratio, rim area, and other optic disc properties in some implementations. HRT may be utilized to assess and monitor glaucoma in some of these embodiments.

Tonometer-a tonometer or the like, such as an Applation Goldman Tonometer, Non-Applanation Tonometer, Air-Puff Tonometer, Ocular Response Analyzer, Non-Contact Tonometer, Indentation Shiotz, Tono-Pen, Rebound Tonometer, and/or iCare Tonometer may be incorporated. These and other devices may be employed in certain embodiments to measure intraocular fluid eye pressure or corneal visco-elasticity, for example, to monitor the integrity of the cornea and/or detect certain disorders, such as keratoconus and glaucoma.

### Varying Levels of Automation and Assistance

As previously stated, the eye testing and evaluation system may be implemented using various levels of automation and/or various forms of on-site and/or remote support. In accordance with certain embodiments, the following discusses a number of exemplary implementations with varied degrees of assistance and automation.

For example, in some embodiments, the system (or a portion of it) may be configured to be totally automated, allowing customers to acquire vision and/or eye health examinations at a Checkup Point diagnostic centre (CuPDC) without the aid of others. In some of these embodiments, the Checkup Point diagnostic centre (CuPDC) may use and execute software applications, programs, and routines that are designed to obtain various information from customers via the customer interface (e.g., customer data) and determine one or more vision examination and/or eye health examination tests and procedures to administer to the customers (e.g., based on the customer data). Similarly, when administering one or more tests to a customer, the Checkup Point diagnostic centre (CuPDC) may use and execute software, such as programs that allow the Checkup Point diagnostic centre (CuPDC) to control and operate ophthalmic equipment, devices, and related components, output instructions, questions, testing data, and other information to customers, and/or receive and record responses. After administering the tests and procedures, Checkup Point diagnostic centre (CuPDC) may use various algorithms, metrics, and software to process and analyze the data captured and recorded through the tests, as well as other data (e.g., customer data), in order to automatically evaluate the customers' vision and/or eye health. In some of these embodiments, the Checkup Point diagnostic centre (CuPDC) may also use and run software to automatically generate one or more reports, documents, and the like relating to the customers' vision and/or eye health (e.g., eye-health reports, prescriptions, recommendations, referrals, and the like) that can be output and/or provided to the customers.

In some embodiments, the system is configured to allow on-site operators (e.g., operators, technicians, or assistants at the locations where the Checkup Point diagnostic centre (CuPDC) are located) to facilitate and assist customers with one or more aspects of eye-health examinations, as well as other services, features, and functionality provided by the Checkup Point diagnostic centre (CuPDC). For example, in some of these implementations, on-site operators may simply assist customers with one or more activities associated with using the Checkup Point diagnostic centre (CuPDC), such as registering an account, selecting desired services, printing an eye health report, and forwarding data to third parties. For instance, on-site operators may assist and guide consumers during the administration of various tests and procedures, such as by assisting customers in assuming the proper positions and answering or responding to certain questions or other data output during the testing. On-site operators may give assistance to consumers on an as-needed or as-desired basis in some of these embodiments, such as in response to a customer selecting an option or otherwise indicating a desire for on-site help.

The on-site operators may monitor and/or control the ophthalmic equipment and devices (e.g., through an on-site Operator interface) in certain embodiments, such as during one or more tests and procedures to ensure proper administration, and/or before and after the tests and procedures to initialize, setup, and/or reset the equipment. In certain of these implementations, on-site operators may also (or instead) monitor and control the administration of one or more tests, such as by selecting and/or adjusting the questions or outputs offered to customers depending on previous customer replies and/or test results. In still alternative implementations, one or more remote operators may offer some or all of the assistance and functionality associated with the on-site operators (e.g., a technician located at a remote call centre or an assistant associated with a remote ophthalmologist).

In some embodiments, the system is configured to allow remote ophthalmologists (e.g., practitioners who are not located at the sites where the Checkup Point diagnostic centre (CuPDC) are located) to facilitate and assist customers with one or more aspects of eye-health examinations and/or other services provided by the Checkup Point diagnostic centre (CuPDC). When a client receives an eye health checkup through a Checkup Point diagnostic centre (CuPDC), for example, various data connected with the consumer, as well as one or more of the tests and treatments conducted to the customer, is provided to an ophthalmologist in some preferred implementations (e.g., via an ophthalmologist device) to enable the ophthalmologist to validate, evaluate, and diagnose the customer's visual capacity and/or eye health, as well as to provide a vision and/or eye health report (or equivalent evaluation) for the client

This process may occur asynchronously in certain of these embodiments. To put it another way, after the customer has received the vision and/or eye health examination, the system may deliver the customer data and examination data to the ophthalmologist (or make such data available to the ophthalmologist through a web-based service and/or software application installed on the ophthalmologist device). After reviewing and analysing the data, the ophthalmologist may diagnose and validate various elements of the customer's vision and eye health and generate and/or send back customer evaluation data at a later time (e.g., vision report, eye health report, optical prescriptions, etc.). This can then be sent to the consumer or made available to them (e.g., at a Checkup Point diagnostic centre (CuPDC), at a device associated with the customer, etc.).

In certain other examples, remote ophthalmologists may provide assistance in real time. In certain of these implementations, the system may be designed to create a real-time connection to a distant practitioner when a customer orders a vision examination and/or eye health examination through a Checkup Point diagnostic centre (CuPDC) (or at any point during the examination or examination process). As a result, the ophthalmologist can study and assess the customer's examination data in real-time or near real-time and deliver evaluations and reports to the customer. Alternatively, the ophthalmologist may be able to monitor and control ophthalmic equipment and instruments, as well as the administration of tests and procedures to the customer, in the same way that the on-site (or remote) operator can, via a real-time connection with the Checkup Point diagnostic centre (CuPDC).

For example, some or all of the ophthalmic equipment and instruments at the Checkup Point diagnostic centre (CuPDC) may be connected to a personal computer running any suitable operating system and containing a client application that provides an interface to (e.g., sends commands to and receives data from) the equipment in certain embodiments. Any relevant application capable of providing a remote interface to the ophthalmic equipment can be used as the client application. In certain examples, the client application may store different data, such as data received from ophthalmic equipment and/or customers (e.g., in a database maintained in local memory at the Checkup Point diagnostic centre (CuPDC)).

The client application and/or personal computer may be connected to a tele-presence system in some implementations, which can be any acceptable system for establishing a real-time connection with, and routing or streaming different data to and from a remote agent (e.g., an ophthalmologist via an ophthalmologist device). The client application and/or personal computer, for example, could be linked to a tele-presence client framework or endpoint.

The tele-presence server may be designed to route the examination and/or examination session to an appropriate tele-presence client framework or endpoint associated with an available distant practitioner in certain embodiments (e.g., an endpoint integrated with or connected to an ophthalmologist device). As a result, data from the examination and/or examination session can be streamed or otherwise given in real-time to the distant practitioner. The endpoint associated with the ophthalmologist may be designed to send different data back to the endpoint associated with the Checkup Point diagnostic centre (CuPDC) in some of these examples. As a result, the ophthalmologist and the customer can communicate in both directions. For example, the ophthalmologist may send back commands for controlling ophthalmic equipment and/or administering tests, audio/video data to be output to the customer (e.g., instructions, questions, guidance, etc.) and/or evaluation data pertaining to the customer's vision and/or eye health (e.g., Vision and/or eye health reports, prescriptions, recommendations, referrals, etc.).

In some examples, the tele-presence system may allow for real-time teleconferencing and video conferencing without the use of specialized networks, endpoints, or other gear (e.g., dedicated cameras, microphones, monitors, firmware, etc.). A centralized teleconferencing infrastructure, for example, may be used by the system to allow and manage conferencing between software endpoints (e.g. devices having certain software installed). This centralized conferencing architecture may be offered and controlled by a separate service in some embodiments.

### Eye Health Evaluations

As previously stated, when a customer receives a vision examination and/or an eye health examination from a Checkup Point diagnostic centre (CuPDC), the Checkup Point diagnostic centre (CuPDC) and/or eye testing and evaluation system may generate and/or provide the customer with customer evaluation data pertaining to the eye health, such as one or more reports, charts, or documents. Some or all of the customer assessment data may be generated automatically by the Checkup Point diagnostic centre (CuPDC) and/or eye testing and evaluation system in some of these embodiments such as by applying various software and algorithms to, and otherwise processing, data related to administering the tests and procedures to the client and/or other data (e.g., customer data). Alternatively, or in addition, part or all of the customer evaluation data may be obtained (or based on data received) from a remote ophthalmologist, such as in response to the ophthalmologist reviewing and analysing the customer examination data and customer data.

Client evaluation data may include eye health evaluation data in some implementations, such as when a customer has an eye health examination through a Checkup Point diagnostic centre (CuPDC). The eye health evaluation data may include an eye health report in some of these embodiments. The eye health report, for example, might include a variety of facts and information about the customer's eyes, such as if they were deemed to be normal and healthy. In certain of these embodiments, the eye health report may show whether or not the client has particular eye conditions, as well as more information about any such disorder found.

When one or more eye disorders or conditions are found, the eye health evaluation data may indicate whether the customer needs to seek additional evaluation, testing, or treatment through an in-person appointment with an eye health practitioner (e.g., an eye surgeon or other specialist). In other instances, such as when customer examination data is sent to an ophthalmologist for assessment, the need for additional in-person evaluation may be determined by the remote ophthalmologist's judgments or suggestions. Alternatively, the system might be set up to automatically detect and/or flag specific examination data or results as indicating a need for the customer to seek additional examination. The eye health evaluation data may contain referrals for the customer to see a subspecialized ophthalmologists in some embodiments. The eye health report in some of these embodiments may indicate that the referral is urgent and/or required, such as to avoid or lessen the risk of permanent vision loss.

The eye health evaluation data may contain one or more suggestions for the client regarding future eye health examinations in some embodiments. For example, the results of an eye health evaluation may imply a recommendation for how frequently the customer should have eye health examinations (e.g., once a year, once every two years, etc.). These recommendations issued by ophthalmic societies (e.g., Deutsche Ophthalmologische Gemeinschaft, American Academy of Ophthalmology) may be based on a variety of customer data (e.g., the customer's age, ethnicity, sex, eye health, personal and family medical history, previous examination) in certain implementations. Similarly, the urgency of recommendations may be decided in a similar way in some implementations. In some cases, the eye health evaluation data may additionally include particular diagnoses and/or reflect a customer's real or suspected eye ailment (e.g., whether a customer has healthy eyes or is suspected of having an eye disease, abnormality or other condition).

While the preceding discussion describes some types of information that may be included in customer evaluation data provided to customers who receive eye health examinations through Checkup Point diagnostic centre (CuPDC), it should be understood that the types of information described are intended to be exemplary, not exhaustive, and that various other types of information may be included as well. Similarly, while the eye health evaluation data are detailed separately in the preceding discussion, it should be understood that in other implementations, the eye health evaluation data are combined. Some or all of the data and information related to eye health evaluations may be combined or merged.

Customer evaluation data may be communicated and/or made accessible to customers through various different systems and devices, such as one or more personal computing devices associated with the customers, according to some embodiments. Customer evaluation data, for example, could be delivered via e-mail or any other relevant delivery channel. Customers may also be able to access customer evaluation data from a variety of computing devices, such as by logging into a web-based service or application provided by the Checkup Point diagnostic centre (CuPDC) and/or by using a software application (e.g., client application, mobile app, etc.) installed on their devices. Customer assessment data may be transmitted to the customer and/or one or more third parties automatically or solely in response to a request from the customer in certain implementations. Client assessment data may be communicated to the customer over the phone in some examples. A consumer who wishes to talk with an ophthalmologist, for example, may supply a phone number so that the ophthalmologist can contact them at a convenient time.

### Data Analysis for Diagnosis and Risk Prediction

The Checkup Point diagnostic centre (CuPDC) and/or other components of the eye testing and evaluation system (e.g., CDC servers, database servers, etc.) may store and/or update a wide range of data in connection with providing vision examinations and/or eye health examinations to customers in accordance with certain embodiments, such as those illustrated and described in connection with Figures 1 and 2. Various client data (e.g., background and demographical data, prior vision examination and/or eye examination results, individual and family medical history, and other traits and preferences) may be stored and/or updated by the eye testing and evaluation system. Similarly, the eye testing and evaluation system may store and/or update customer examination data (e.g., customer inputs and responses, instrument measurements, tests and procedures performed, etc. ), customer evaluation data (e.g., reports, recommendations, referrals, digital images and other imaging data, etc., generated by the system and/or received from remote ophthalmologists), and customer evaluation data (e.g., reports, recommendations, referrals, digital images and other imaging data, etc.)

In certain implementations, the eye testing and evaluation system may track and evaluate the customers' eye health over time using some or all of the stored data mentioned above. When a customer has an eye health examination through a Checkup Point diagnostic centre (CuPDC), for example, the examination data and/or evaluation data may be compared to data from previous eye health examinations, and other similar tests (e.g., data associated with previous tests received through a Checkup Point diagnostic centre (CuPDC), from an external ophthalmologist). Using the current and prior examination and/or evaluation data, the eye testing and evaluation system may generate and/or update various charts, graphs, photos, summaries, and the like, such as to show the progression of one or more parameters, defects, disorders, and/or conditions.

As a result, the eye testing and evaluation system can determine whether a treatment previously prescribed to the customer is working (e.g., whether there has been any improvement or change in a defect or condition being treated) and/or evaluate the outcome of a surgery, operation, or procedure previously performed on the customer by tracking their eye health over time. Similarly, the eye testing and evaluation system may use tracking data to detect specific abnormalities, defects, and/or conditions, as well as to determine whether a consumer should be sent to one or more local ophthalmologist.

A customer is first given one or more tests linked with an eye health assessment. One or more tests may be administered at a Checkup Point diagnostic centre (CuPDC) in some implementations. Following that, customer examination data for the eye health examination is created. Any data associated with the customer, as well as any data that enables or assists an ophthalmologist in evaluating the customer's eye health, detecting certain disorders, defects, and conditions, and/or confirming that one or more tests and procedures were administered correctly, may be included in the customer examination data. Data connected with the customer (e.g., the customer's name, age, gender, race, medical history, past test results, etc.) and data associated with one or more of the tests conducted to the customer may be included in certain embodiments of the customer examination data (e.g., responses, inputs and selections from the customer, instrument measurements and readings, test results, etc.). Client examination data may include a live video stream or video recording of an eye health examination supplied to a customer in some implementations.

Customer examination data is sent from a Checkup Point diagnostic centre (CuPDC) to a diagnostic centre server over a computer network (e.g., the Internet). All or part of the customer examination data may be stored on the diagnostic centre server. The diagnostic centre server grants access to one or more practitioner devices, allowing them to view the client examination data. This allows a practitioner using a practitioner device to study customer examination data from a location other than the Checkup Point diagnostic centre (CuPDC) where the one or more tests are given to the customer. In some examples, a practitioner may have real-time access to client examination data as tests are administered to the consumer. In some cases, a practitioner may get access to the client examination data before the tests are given to the customer.

Customer evaluation data is generated after or while the ophthalmologist is examining the customer examination data. Various reports, recommendations, and other information showing the outcomes of the eye health tests and procedures provided to the customer may be included in the customer assessment data obtained from the remote ophthalmologist. Customer assessment data may include an eye health report or be used to produce an eye health report in some embodiments. A summary of the customer's eye health, recommendations, referrals to other local ophthalmologists, and/or other data related to the customer, practitioner, or customer's visit to the Checkup Point diagnostic centre (CuPDC) may be included in the eye health report. The client receives an eye health report that is based, at least in part, on the customer evaluation data over the network. A customer's eye health report can be delivered in a variety of ways. The eye health report may be presented to the consumer through the diagnostic centre in certain embodiments. The customer may be allowed to access the eye health report via one or more computing devices associated with the customer in some embodiments. After logging in (e.g., with a username and password or Q/R code) to an account on the website, the customer may be permitted to download a copy of the eye health report in certain implementations. The eye health report may be sent to the customer by electronic mail or postal service in some implementations.

According to some aspects of the present invention, a synchronous eye health assessment is administered. The connection between a Checkup Point diagnostic centre (CuPDC) and an ophthalmologist device is established first. In some examples, the Checkup Point diagnostic centre (CuPDC) may send a request to a server for connection establishment, and the request may be accepted by the ophthalmologist device. The server or an individual at the Checkup Point diagnostic centre (CuPDC) and the ophthalmologist may choose an ophthalmologist associated with the ophthalmologist device. In some embodiments, the connection may provide a live audio/video stream to both the ophthalmologist device and the Checkup Point diagnostic centre (CuPDC), allowing communication between an ophthalmologist operating the ophthalmologist device and a customer and/or on-site personnel at the Checkup Point diagnostic centre (CuPDC). For security reasons, all data exchanged over the connection may be encrypted.

For the purpose of conducting the eye health examination, an ophthalmologist may be allowed to control one or more ophthalmic devices placed at the client diagnostic centre. As previously stated, the ophthalmologist device may have an interface that allows the distant practitioner to manage and manipulate the equipment by selecting interface elements or in other ways. Any equipment relevant to performing an eye health examination, including but not limited to a digital imager, bio-microscope, fundus camera, tonometer, may be controlled by the remote ophthalmologist. The eye health examination may then be administered to the customer at the Checkup Point diagnostic centre (CuPDC) while the ophthalmologist monitors the eye health examination in real-time.

The saved examination data on the server may be made available to one or more remote ophthalmologists. In some examples, the remote ophthalmologists may receive and accept a request from the customer to do the examination. An individual at the Checkup Point diagnostic centre (CuPDC) or the server can choose the remote ophthalmologists. To access the stored examination data connected with the customer, the ophthalmologist may enter login credentials (e.g., username and password) in certain implementations. The ophthalmologist may send evaluation data and/or an eye health report to the consumer after analysing the examination data. As previously stated, the evaluation data and/or an eye health report may be provided to the customer while the customer is at the customer diagnostic centre or afterwards by other means.

It should be noted that the procedures described above are only intended to show how vision and eye health screenings can be administered to consumers and are not intended to be exhaustive. The methods can be modified in a variety of ways without deviating from the scope of the present invention.

Through the Checkup Point diagnostic centre (CuPDC) and/or the eye testing and assessment System, consumers may be able to see, compare, order, and/or purchase various eye care related items and services. For example, the Checkup Point diagnostic centre (CuPDC) and/or the eye testing and assessment system may store and/or access various information connected to items sold by one or more third parties (e.g., eye related vitamin selections etc.). Customers may be able to access, view, and/or browse these products at Checkup Point diagnostic centre (CuPDC) as a result (e.g., through a display screen included with the customer interface).

The Checkup Point diagnostic centre (CuPDC) and/or the eye testing and assessment system may be configured to recommend eye care related items and services to customers in certain implementations. For example, based on different data connected with the customer, such as customer data (e.g., income, sex, age, prior products purchased, etc.) and/or customer examination and assessment data, the eye testing and evaluation system may recommend one or more products to the client (e.g. retinal fundus findings, etc.). Similarly, the eye testing and evaluation system may make product recommendations for a consumer based on information about other customers, such as things purchased by customers with similar features, backgrounds, or problems. Product suggestions in some other embodiments may be based on a "preferred" status associated with one or more third-party vendors (e.g., vendors who pay a fee to emphasize their products and services).

In certain implementations, Checkup Point diagnostic centre (CuPDC) may give various advertisements and/or promotional materials (e.g., coupons, offers, discounts, etc.) to customers. Advertisements and promotions may be displayed to consumers (e.g., web shop) before, during, and/or after the client has an eye health examination through the Checkup Point diagnostic centre (CuPDC) in some of these implementations. The advertising and promotional materials may comprise materials linked with a variety of eye care-related products and services, as well as materials for a wide range of other consumer products and services in certain embodiments.

Customers gain from the incorporation of advertising and promotional materials, as well as other related services (e.g., allowing suppliers to pay for preferred status). Customers may be able to receive vision examinations and/or eye health examinations at a significantly reduced cost, or even receive certain services free of charge, or even receive coupons for family and friends, as a result of the revenue generated through such mechanisms, encouraging customers to receive such examinations on a regular basis and recommend them to others.

In some cases, the system can be set up to send customers various notifications and messages (e.g., through email, text). For example, the eye testing and assessment system may send a message to a customer to remind the customer that it is time for an eye health examination follow-up, or to remind the customer about an approaching appointment with an external ophthalmologist in certain implementations (e.g., in connection with a previous referral for in-person evaluation). Similarly, the eye testing and evaluation system may send a client a message requesting confirmation that the customer has scheduled and/or seen an external practitioner (e.g., in the case where a referral for urgent follow-up testing was provided to the customer).

It should be noted that the various systems, devices, and methods mentioned in connection with the preceding figures are merely examples, and that any other acceptable systems, devices, or methods may be employed instead. The preceding is only illustrative of the invention's principles, and those versed in the art can make different variations without straying from the extent and spirit of the invention.

### Artificial intelligence assisted solution

Medical imaging, such as retinal imaging, is used to diagnose many ocular diseases and disorders. Human professionals have typically analysed images separately in medical imaging. The demand for efficient and precise image analysis is outstripping the supply of experts capable of performing this role as the number of medical imaging procedures grows. The imaging of human organs is crucial in the diagnosis of a variety of disorders. This is especially true for the human retina, which has a massive network of blood arteries and neurons, making it a near-ideal window for studying the impact of disorders that affect vision (such as diabetic retinopathy, macular degeneration, glaucoma, and so on) as well as other systemic diseases (hypertension, stroke, and so forth). Although certain embodiments of the eye testing and evaluation system have been described in relation to a specific number of Checkup Point diagnostic centre (CuPDC) and/or ophthalmologist devices with specific formats or types, the platform may include any number, formal, and type of Checkup Point diagnostic centre (CuPDC) and/or ophthalmologist devices. While the Checkup Point diagnostic centre (CuPDC) have been characterized as having certain exemplary ophthalmic equipment and instruments, they may use almost any suitable sort of such devices and instruments.

### Triage:

An identification of a normal fundus image with the aid of artificial intelligence is desirable in order to reliably distinguish a normal finding from a pathological finding. About 85% of the fundus images when screening a normal population show no abnormal changes. Reliable detection of a normal fundus image would therefore greatly reduce the workload of the ophthalmologist, so that only the remaining 15% have to be subjected to an ophthalmological assessment.

To make imaging-based illness detection scalable, cost-effective, and repeatable, advances in computer-aided image processing and analysis technologies are required. Such advancements would directly result in effective patient triage, resulting in prompt treatment. One embodiment of such an approach could be an anomaly detection system, which takes as an input a set of retinal fundus images, scalar measurement data such as the intraocular pressure, refraction, body-mass-index or other clinical data. The multimodal data is processed using an artificial neural network und provides as an output a probability of anomaly, i.e. pathology for the given case. Based on that probability, the software system can flag the case as normal or refer to the case to a remote ophthalmologist to inspect it manually.

In another embodiment, the system may take as an input a set of retinal fundus images, scalar measurement data such as the intraocular pressure, refraction, body-mass-index or other clinical data. The multimodal data is then processed using an artificial neural network to provide a probability for the presence of a given set of pathologies, e.g. diabetic retinopathy, age-related macula degeneration and glaucoma. Based on these probabilities, the software system can flag the case as normal or refer to the case to a remote ophthalmologist to inspect it manually.

These two approaches can also be combined in an ensemble, combining the probabilities of pathologies from both approaches for improved performance of detecting normal cases.

### Diagnosis:

In a next step it might be desirable to identify the major fundus pathologies like diabetic retinopathy, age related macular degeneration and glaucoma just to mention a few. To this end, the cases that were either flagged as abnormal by the triage algorithm or manually inspected by an ophthalmologist are automatically analysed using an artificial neural network for disease classification. Given multimodal input data including one or more fundus images and measurement data such as intraocular pressure and refraction, an embodiment could predict probabilities for the presence for a given set of pathologies. These predictions could either be displayed to an ophthalmologist as suggestions or used to make a fully autonomous diagnosis if the confidence exceeds a certain threshold.

### Prognosis:

In a later stage it might be desirable to give an estimate based on early fundus findings if the certain individual is at risk in developing a heart failure, stroke, dementia and or other systemic diseases which can first be identified by distinct pathological changes on the human retina. To this end, an embodiment may provide a risk score for cardiovascular or neurodegenerative pathological changes given a set of fundus images and clinical data such as intraocular pressure, body mass index and age.

## Claims

1. A system for providing eye health examinations, comprising:
* a plurality of diagnostic centres, wherein the diagnostic centres are configured to:
- transmit requests to a network of ophthalmologist to administer eye examinations for customers;
- receive instructions over the network to remotely control operation of ophthalmic equipment in order to administer one or more tests pertaining to the eye examinations; and
- generate customer examination data pertaining to the one or more tests administered using the ophthalmic equipment;
* a plurality of practitioner devices associated with ophthalmologists, wherein the practitioner devices are configured to:
- receive the customer examination data in response to accepting one or more of the requests associated with administering the eye examinations;
- display the received customer examination data; and
- generate customer evaluation data associated with the one or more accepted requests;
* at least one server configured to:
- in response to receiving the requests from the diagnostic centres, analyse practitioner data to select ophthalmologists for handling the requests. wherein the practitioner data includes one or more of the following:
+ availability data indicating whether the ophthalmologists are currently connected to the system and available to administer eye examinations;
+ scheduling data indicating days and hours that the ophthalmologists are available to handle the requests; and
+ prior customer data that identifies customers who utilized the ophthalmologists for prior eye examinations;
- receive the customer examination data from the diagnostic centres;
- transmit the customer examination data to the practitioners devices associated with the selected ophthalmologists;
- receive customer evaluation data from the practitioner devices associated with the selected ophthalmologists;
wherein eye health reports based, at least in part, on the selected ophthalmologists' review and evaluation of the customer examination data are provided to the cus-tomers.

2. The system of claim 1, wherein the at least one server is integrated with the Checkup Point diagnostic centre (CuPDC).

3. The system of claim 1 or 2, wherein the requests include requests pertaining to synchronous eye health examinations, and the at least one server is further configured to:
- establish real-time connections between the practitioner devices associated with the selected ophthalmologists and the diagnostic centres to permit communications between the customers and the selected ophthalmologists during the synchronous eye health examinations;
- permit the practitioner devices associated with the selected ophthalmologists to remotely administer one or more tests associated with the synchronous eye health examinations over the network via the real-time connections, wherein the instructions received by the diagnostic centres over the network are utilized to control the ophthalmic equipment during the synchronous eye health examinations; and
- receive customer evaluation data associated with the one or more tests from the practitioner devices associated with the selected ophthalmologists while the synchro-nous eye health examinations are being administered;
wherein the eye health reports provided to the customers includes at least a portion of the customer evaluation data associated with the one or more tests.

4. The system of any one of the preceding claims, wherein the requests include requests pertaining to asynchronous eye health examinations, and the at least one server is further configured to:
- receive customer examination data generated by the ophthalmic equipment at the diagnostic centres which pertains to the asynchronous eye health examinations; and
- store the customer examination data on the at least one server, wherein the selected ophthalmologists are provided access to the customer examination data after the asynchronous eye health examinations are administered;
wherein the eye health report received at the diagnostic centre includes the selected eye-care practitioner's assessment of the customer examination data pertaining to the asynchronous eye health examination.

5. The system of any one of the preceding claims, wherein the ophthalmic equipment comprises eye health equipment that enables customers to receive comprehensive eye health examinations at the Checkup Point diagnostic centre (CuPDC), the eye health equipment including at least one tonometer and digital imager.

6. The system of any one of the preceding claims, wherein the at least one server is further configured to generate aggregate result data based on an analysis of stored customer examination data for the customers.

7. The system of any one of the preceding claims, wherein the aggregate result data identifies one or more risk factors pertaining to groups of individuals with a higher risk of having a particular eye health disorder.

8. The system of any one of the preceding claims, wherein the at least one server selects the ophthalmologists, at least in part, by analysing the availability data to identify available eye-care practitioner devices that are currently connected to the system and which are available to handle the requests.

9. The system of any one of the preceding claims, wherein the at least one server selects the ophthalmologists, at least in part, by analysing the prior customer data and identifying ophthalmologists who were previously utilized by the customers for one or more prior eye examinations.

10. The system of any one of the preceding claims, wherein the at least one server selects the ophthalmologists, at least in part, by utilizing the scheduling data to determine whether the ophthalmologists are currently available.

11. A server for providing for providing services related to eye health examinations, comprising:
- receiving a request over a network from a diagnostic centre for selecting an eye-care practitioner to administer an eye examination to a customer;
- in response to receiving the request, analysing practitioner data to select the eye-care practitioner for handling the request, wherein the practitioner data includes one or more of the following:
+ availability data indicating which ophthalmologists are currently available to administer the eye examination;
+ scheduling data indicating days and hours that ophthalmologists are available to handle requests; and
+ prior customer data that identifies one or more ophthalmologists who previously administered eye examinations to the customer;
- receiving customer examination data generated by ophthalmic equipment at the diagnostic centre while administering one or more tests pertaining to the eye examination;
- transmitting customer examination data pertaining to the one or more tests to the practitioner device associated with the selected eye-care practitioner;
- receiving customer evaluation data from the practitioner device associated with the selected eye-care practitioner;
wherein an eye health report based, at least in part, on the selected eye-care practitioner's review and evaluation of the customer examination data is provided to the customer.

12. The server of claim 11, wherein the at least one server is integrated with the Checkup Point diagnostic centre (CuPDC).

13. The server of claims 11 or 12, wherein the request pertains to a synchronous eye health examination, and the server is further configured to:
- establish a real-time connection between the practitioner device associated with the selected eye-care practitioner and the diagnostic centre to permit communications between the customer and the selected eye-care practitioner during the synchronous eye health examinations;
- permit the practitioner device associated with the selected eye-care practitioner to remotely administer one or more tests associated with the synchronous eye health examination over the network via the real-time connection, wherein instructions received by the diagnostic centre over the network are utilized to control the ophthalmic equipment during the synchronous eye health examination; and
- receive customer evaluation data associated with the one or more tests from the practitioner device associated with the selected eye-care practitioner while the synchronous eye health examination is being administered, wherein the eye health report provided to the customer includes at least a portion of the customer evaluation data associated with the one or more tests.

14. The server of any one of the preceding claims 11 to 13, wherein the request pertains to an asynchronous eye health examination, and the server is further configured to:
- receive customer examination data generated by the ophthalmic equipment at the diagnostic centre which pertains to the asynchronous eye health examination; and
- store the customer examination data on the server, wherein the selected eye-care practitioner is provided access to the customer examination data after one or more tests associated with the asynchronous eye health examination are administered;
wherein the eye health report provided to the customer includes the selected eye-care practitioner's assessment of the customer examination data pertaining to the asynchronous eye health examination.
